# EUROPEAN PATENT APPLICATION

(11) **EP 2 264 454 A1**
(43) Date of publication of application: **22.12.2010**
(21) Application number: 09162498.1
(22) Date of filing: 11.06.2009
(51) Int. Cl.: G01N 33/50

(54) **Method for screening compounds for preventing the adverse effects of reactive oxygen species on eukaryotic cells**

(71) Applicant: Universiteit Maastricht, 6211 LK Maastricht (NL)
(72) Inventor: Kleinjans, Joseph Catharina Stephanus, 6221 AR Maastricht (NL); Briedé, Jacob Jan, 6241 CZ Bunde (NL)
(74) Representative: Habets, Winand

(57) **Abstract**

The invention is in the field of molecular medicine, more in particular in the field of identifying new compounds for the treatment or prevention of diseases as well as ageing. The invention provides a method for the screening and/or identification of compounds that are capable of preventing or ameliorating the adverse effects of reactive oxygen species on eukaryotic cells. More in particular, the invention provides a method for the identification of an active therapeutic compound for preventing or ameliorating the adverse effects of reactive oxygen species on eukaryotic cells comprising the steps of:providing an assay wherein the expression of a pathway can be determined under the influence of a reactive oxygen species, providing a candidate therapeutic compound, determining the ability of said therapeutic compound to normalize the expression of said gene in the presence of said oxidant, wherein said pathway is selected from the group consisting of pathways as disclosed herein

## Description

### Field of the invention

The invention is in the field of molecular medicine, more in particular in the field of identifying new compounds for the treatment or prevention of diseases. The invention provides a method for the screening and/or identification of compounds that are capable of preventing or ameliorating the adverse effects of reactive oxygen species on eukaryotic cells.

### Background of the invention

The balance between oxidant and antioxidant activities determines the extent of oxidative damage induced by reactive oxygen species (ROS) in cells or tissues. Oxidative stress may occur in almost any tissue, but particularly the imbalance between high oxidant exposure and antioxidant capacity in target tissue for disease and ageing has been linked to increased health risks [1-3]. For instance, patients with inflammatory bowel diseases, accompanied by oxidative stress [4], are at increased risk for developing colorectal cancer [5].

The most relevant forms of reactive oxygen species, namely the hydroxyl- (HO.) and superoxide anion (02.-) radicals, have preferred cellular targets and react with different kinetics with antioxidants enzymes [6]. Low levels of oxidative stress activate the transcription of genes encoding proteins that participate in the defence against oxidative injuries, oxidative damage repair mechanisms and apoptosis. [7]. In the early response to increased oxidative stress in mammalian cells the nuclear factor (erythroid-derived 2)-like 2 (NFE2L2 or NRF2) represents a crucial sensor [7]. After nuclear accumulation of NRF2 [8,9] transcriptional activation via the antioxidant responsive element (ARE) results in the induction of antioxidants enzymes including the (SOD1, 2 and 3), catalase (CAT) and glutathione peroxidase (GPx).

Also, the small G-protein RAS is an upstream signalling element during oxidative stress. It activates activator protein 1 (AP-1) and nuclear factor kappa B (NF-kappaB) which in turn are involved in the activation of other antioxidant genes including thioredoxin (TRX) [10]. In case the first line of defence against oxidative damage is insufficient to prevent the induction of DNA damage, several other (signalling) processes are initiated. First, the cells can repair the oxidative DNA damage by base excision repair (BER) [11]. Further, in order to allow more time for repair of damages, cell proliferation can be blocked at several phases of the cell cycle, such as G1-S transition, S-phase and G2-M transition [12].

Methods for the identification of compounds that influence the adverse effects of reactive oxygen species on eukaryotic cells have been described in the prior art. However, so far, no reliable methods have been described that are suitable for the identification of compounds on a large scale and/or on a high-throughput scale.

### Summary of the invention

We herein present a method for the identification of an active therapeutic compound for preventing or ameliorating the adverse effects of reactive oxygen species on eukaryotic cells comprising the steps of:
a) providing an assay wherein the expression of a pathway can be determined under the influence of a reactive oxygen species,
b) providing a candidate therapeutic compound,
c) determining the ability of said therapeutic compound to normalize the expression of said pathway after exposure to the reactive oxygen species or even in the presence of the reactive oxygen species,
   wherein said pathway is selected from the group consisting of Arginine metabolism, Polyamine metabolism, (L)-Arginine metabolism, IAP-proteins in apoptosis, regulation of aminoacid metabolism, WNT signaling pathway, Chromosome condensation in prometaphase, Urea cycle, ECM remodeling, Glucocorticoid receptor signaling, Initiation of mitosis, Aspartate and asparagine metabolism, Role of Akt in , hypoxia induced HIF1 activation, IL-3 activation and signaling pathway, IL-2 activation , and signaling pathway, Activin A signaling regulation, Vitamin B7 (biotin) metabolism, Chromosome condensation in prometaphase, NOTCH1-mediated pathway for NF-KB activity modulation, Start of DNA replication in early S phase, ATM/ATR regulation of G1/S checkpoint, , Leucine, isoleucine and valine metabolism, Role of SCF complex in cell cycle regulation, Propionate metabolism, Sin3 and NuRD in transcription regulation, Brca1 as a transcription regulator, Spindle assembly and chromosome separation, Sister chromatid cohesion, Glutathione metabolism, Notch Signaling Pathway, ESR1 regulation of G1/S transition, P53 signaling pathway, Transition and termination of DNA replication, Heme metabolism, Initiation of mitosis, Triacylglycerol metabolism ,Bile Acid Biosynthesis and Role of Akt in hypoxia induced HIF1 activa.

### Detailed description of the invention

Herein we compare global gene expression changes in human colon carcinoma (Caco-2) cells, as an in vitro model for the target organ in vivo, by extensive time series analyses using DNA microarrays following exposure to H2O2 or menadione, leading to the formation of HO. or 02.- radicals, respectively.

In order to ascertain intracellular radical dose, electron spin (ESR) resonance analysis was performed. To associate gene expression modification with phenotypic markers of oxidative stress, whole genome gene expressions were related to oxidative DNA damage (8-oxodG) levels as well as apoptosis and cell cycle progression

Caco-2 cells pre-treated with the spin trap DMPO were exposed to H2O2 or menadione and analysed with ESR spectroscopy for cellular radical formation. Increasing concentrations of H2O2 resulted in dose-dependent formation of DMPO.-OH (data not shown). Based on the detected increase in cellular HO. formation in combination with the absence of the induction of apoptosis a non-cytotoxic concentration of 20 uM H2O2 was chosen. The absence of apoptosis is crucial factor in our experimental set-up, because this would subsequently result in loss of cells from the investigated cell populations. Next, it was detected by ESR that increasing concentrations of menadione showed an 02.--derived DMPO.-OH signal at 100 uM and 45 minutes incubation time, in combination with the absence of apoptosis, so this concentration was selected.

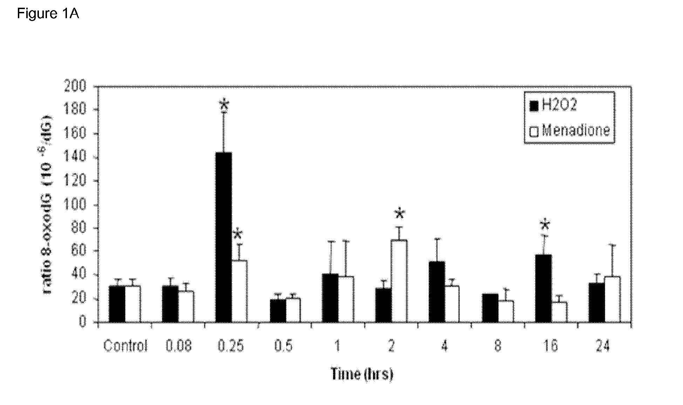

Compared to the constant basal level (20.10-6 8-oxodG/dG) in control cells, H2O2 resulted in an immediately significant increased peak in 8-oxodG at 15 minutes that rapidly decreased to baseline level followed by a smaller but again significantly elevated level at 16 hours after exposure (Fig. 1A). In contrast, the highest significantly increased level of 8-oxodG after menadione exposure was found at 2 hours, while the significant increased level detected at 15 minutes was much lower as compared to H2O2 exposure at 15 minutes.

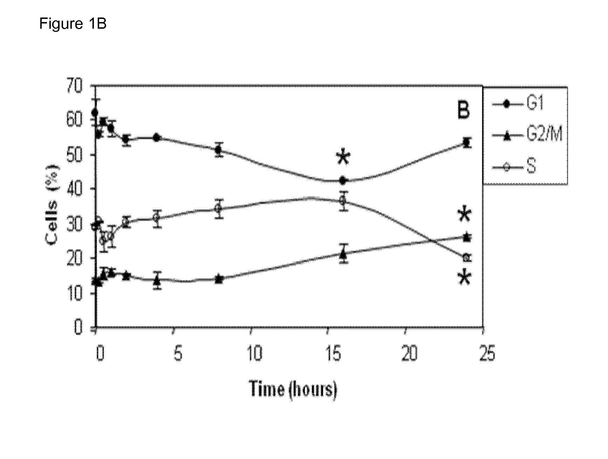

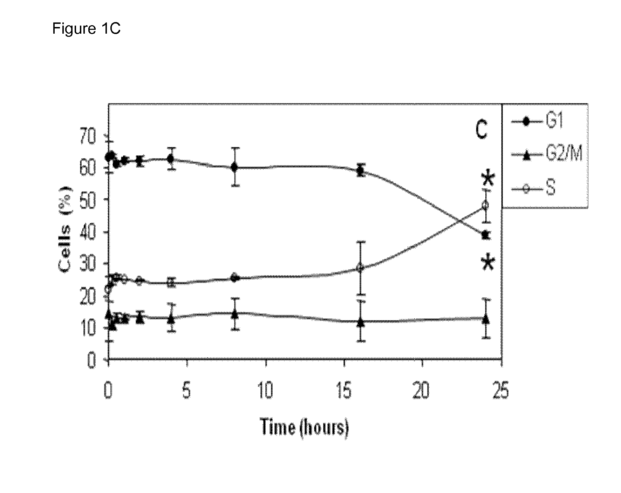

Analyses of cell cycle distributions showed that 16 h after H2O2 exposure, the number of cells in G1 phase significantly decreased as compared to control cells (Fig. 1 B). At 24 h, the percentage of cells in the S phase significantly decreased and in the G1/M phase significantly increased, reflecting progression of cells previously in cell cycle arrest. After menadione exposure (Fig. 1 C) at 2A h a significant increase in the number of cells in the S phase, concomitantly with a significant decrease of cells in the G1 phase was detected. This suggests an arrest of cells in S-phase and/or a stimulation of G1 cells to go into S phase at 24 h. Altogether, this indicates that cell cycle arrest took place much later in menadione-exposed Caco-2 cells than in H2O2-exposed cells.

From these experiments we conclude that both H2O2 and menadione exposure affect the cell cycle progression of Caco-2 cells although this response towards oxidative stress is induced later in superoxide-exposed cells than in hydroxyl radical exposed cells.

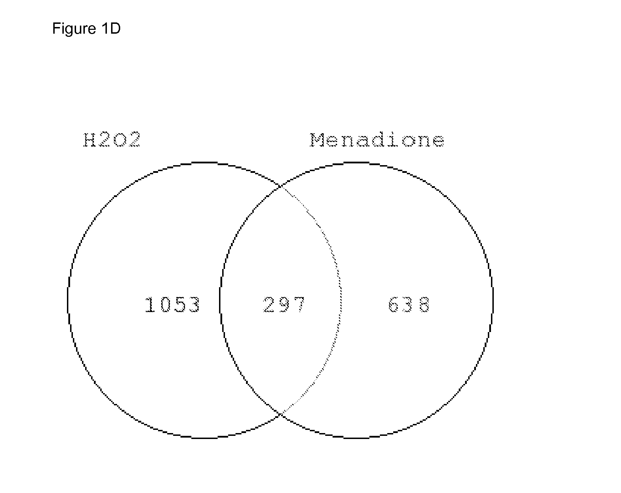

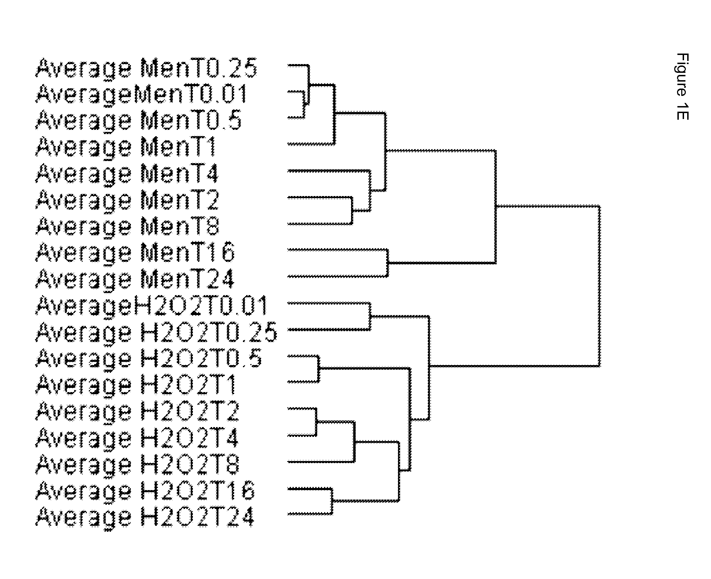

Gene expression analysis resulted in 1404 differentially expressed genes upon H2O2 challenge and 979 genes after menadione treatment. Visualisation of the expression ratios and all arrays in a heat map shows a high reproducibility in the expression profiles between the various replicate experiments and arrays (Fig. 1). In total 297 genes appeared commonly modified after both oxidant exposures (Fig. 1 D), including the antioxidant enzyme CAT (catalase).

Pathway analysis (Table 1) indicated that the significant regulated pathways are vitamin B7 and PPAR regulation of metabolism, induction of transcription via HIF1 activation and other apoptosis pathways and the WNT signaling pathway. The significant pathways exclusively regulated in the gene set induced by H2O2 were aminoacid metabolism and the immune response pathway oncostatin M signalling via MAPK.

**Table 1: Table H2O2: Significant pathways (p<0.01) and genes induced by hydroxyl radical formation.**

| **Pathway** | **Gene Symbol** | **Gene description** |
|---|---|---|
| Arginine metabolism | ALDH1B1 | Aldehyde dehydrogenase X, mitochondrial |
| | AMD1 | S-adenosylmethionine decarboxylase |
| | ARG1 | proenzyme |
| | CKB | Arginase-1 |
| | CPS1 | Creatine kinase B-type |
| | GATM | Carbamoyl-phosphate synthase [ammonia], |
| | LOXL1 | mitochondrial |
| | ODC1 | Glycine amidinotransferase, mitochondrial |
| | RARS | Lysyl oxidase homolog 1 |
| | SAT1 | Ornithine decarboxylase |
| | SAT2 | Arginyl-tRNA synthetase, cytoplasmic |
| | SRM | Diamine acetyltransferase 1 |
| | | Diamine acetyltransferase 2 |
| | | Spermidine synthase |
| Polyamine metabolism | ALOH1A1 | Retinal dehydrogenase 1 |
| | AMD1 | S-adenosylmethionine decarboxylase |
| | GATM | proenzyme |
| | ODC1 | Glycine amidinotransferase, mitochondrial |
| | SAT1 | Ornithine decarboxylase |
| | SAT2 | Diamine acetyltransferase 1 |
| | SRM | Diamine acetyltransferase 2 |
| | | Spermidine synthase |
| (L)-Arginine metabolism | ALDH1B1 | Aldehyde dehydrogenase X, mitochondrial |
| | AMD1 | S-adenosylmethionine decarboxylase |
| | CKB | proenzyme |
| | CPS1 | Creatine kinase B-type |
| | GATM | Carbamoyl-phosphate synthase [ammonia], |
| | ODC1 | mitochondrial |
| | RARS | Glycine amidinotransferase, mitochondrial |
| | SAT1 | Ornithine decarboxylase |
| | SAT2 | Arginyl-tRNA synthetase, cytoplasmic |
| | SRM | Diamine acetyltransferase 1 |
| | | Diamine acetyltransferase 2 |
| | | Spermidine synthase |
| Apoptosis and survival: | CASP3 | Caspase-3 |
| Role of IAP-proteins in | CASP6 | Caspase-6 |
| apoptosis | CDC2 | G2/mitotic-specific cyclin-B1 |
| | HSPA14 | Heat shock 70 kDa protein 14 |
| | HSPA1A | Heat shock 70 kDa protein 1 |
| | HSPA1B | Heat shock 70 kDa protein 1 |
| | HSPA2 | Heat shock-related 70 kDa protein 2 |
| | NAIP | Baculoviral IAP repeat-containing protein 1 |
| | TRADD | Tumor necrosis factor receptor type 1-associated DEATH |
| Transcription:Transcription | FOSB | Protein fosB |
| regulation of aminoacid | JUN | Transcription factor AP-1 |
| metabolism | JUNB | Transcription factor jun-B |
| | MAFG | Transcription factor MafG |
| | MAX | Protein Max |
| | MYC | Myc proto-oncogene protein |
| | NFE2L2 | Nuclear factor erythroid 2-related factor 1 |
| | ODC1 | Ornithine decarboxylase |
| | PRKCH | Protein kinase C eta type |
| | ZNF148 | Zinc finger protein 148 |
| Development: WNT | BIRC5 | Baculoviral IAP repeat-containing protein 5 |
| signaling pathway | CD44 | CD44 antigen |
| | DKK1 | Dickkopf-related protein 1 |
| | ENC1 | Ectoderm-neural cortex protein 1 |
| | FZD4 | Frizzled-4 |
| | FZD5 | Frizzled-5 |
| | FZD6 | Frizzled-6 |
| | FZD7 | Frizzled-7 |
| | JUN | Transcription factor AP-1 |
| | MMP26 | Matrix metalloproteinase-26 |
| | MYC | Myc proto-oncogene protein |
| | RUVBL2 | RuvB-like 2 |
| | WNT5A | Protein Wnt-5a |
| | WNT6 | Protein Wnt-6 |
| Cell cycle: Chromosome | AURKB | Serine/threonine-protein kinase 12 |
| condensation in | CCNB1 | G2/mitotic-specific cyclin-B1 |
| prometaphase | CCNB2 | G2/mitotic-specific cyclin-B2 |
| | CDC2 | Cell division control protein 2 homolog |
| | H1F0 | Histone H1.0 |
| | INCENP | Inner centromere protein |
| | NCAPD2 | Condensin complex subunit 1 |
| | TOP1 | DNA topoisomerase 1 |
| Urea cycle | ARG1 | Arginase-1 |
| | CKB | Creatine kinase B-type |
| | CPS1 | Carbamoyl-phosphate synthase [ammonia], |
| | FH | mitochondrial |
| | GAMT | Fumarate hydratase, mitochondrial |
| | GATM | Guanidinoacetate N-methyltransferase |
| | OAT | Glycine amidinotransferase, mitochondrial |
| | ODC1 | Ornithine aminotransferase, mitochondrial |
| | SRM | Ornithine decarboxylase |
| | | Spermidine synthase |
| Cell adhesion: ECM | CD44 | CD44 antigen |
| remodeling | COL4A6 | Collagen alpha-6(IV) chain |
| | EZR | Ezrin |
| | ITGB1 | Integrin beta-1 |
| | KLK2 | Kallikrein-2 |
| | LAMB3 | Laminin subunit beta-3 |
| | LAMC1 | Laminin subunit gamma-1 |
| | LAMC2 | Laminin subunit gamma-2 |
| | MMP1 | Interstitial collagenase |
| | MMP13 | Collagenase 3 |
| | SERPINE2 | Glia-derived nexin |
| | TIMP2 | Metalloproteinase inhibitor 2 |
| | VTN | Vitronectin |
| Development: | CEBPB | CCAAT/enhancer-binding protein beta |
| Glucocorticoid | HSP90AA1 | Heat shock protein HSP 90-alpha |
| receptor signaling | HSPA14 | Heat shock 70 kDa protein 14 |
| | HSPA1A | Heat shock 70 kDa protein 1 |
| | HSPA1B | Heat shock 70 kDa protein 1 |
| | HSPA2 | Heat shock-related 70 kDa protein 2 |
| | JUN | Transcription factor AP-1 |
| | MMP13 | Collagenase 3 |
| | NR3C1 | Glucocorticoid receptor |
| | RELB | Transcription factor ReIB |
| Cell cycle: Initiation of | CCNB1 | G2/mitotic-specific cyclin-B1 |
| mitosis | CCNB2 | G2/mitotic-specific cyclin-B2 |
| | CDC16 | Cell division cycle protein 16 homolog |
| | CDC2 | Cell division control protein 2 homolog |
| | CDC25C | M-phase inducer phosphatase 3 |
| | H1F0 | Histone H1.0 |
| | LMNB2 | Lamin-B2 |
| | MYC | Myc proto-oncogene protein |
| | NCL | Nucleolin |
| Aspartate and asparagine | ADSS | Adenylosuccinate synthetase isozyme 2 |
| metabolism | ADSSL1 | Adenylosuccinate synthetase isozyme 1 |
| | CPS1 | Carbamoyl-phosphate synthase [ammonia], |
| | DARS | mitochondrial |
| | GAD1 | Aspartyl-tRNA synthetase, cytoplasmic |
| | NARS | Glutamate decarboxylase 1 |
| | | Asparaginyl-tRNA synthetase, cytoplasmic |
| Transcription: Role of Akt in | BNIP3 | BCL2/adenovirus E1 B 19 kDa protein- |
| hypoxia induced HIF1 | CCT2 | interacting protein 3 |
| activation | CCT5 | T-complex protein 1 subunit beta |
| | CCT6A | T-complex protein 1 subunit epsilon |
| | HSPA14 | T-complex protein 1 subunit zeta |
| | HSPA1A | Heat shock 70 kDa protein 14 |
| | HSPA1B | Heat shock 70 kDa protein 1 |
| | HSPA2 | Heat shock 70 kDa protein 1 |
| | SLC2A1 | Heat shock-related 70 kDa protein 2 |
| | | Solute carrier family 2, facilitated glucose |
| | TF | transporter member 1 |
| | | Serotransferrin |
| Immune response: IL-3 | CISH | Cytokine-inducible SH2-containing protein |
| activation | EGR1 | Early growth response protein 1 |
| and signaling pathway | FOSB | Protein fosB |
| | ID1 | DNA-binding protein inhibitor ID-1 |
| | JUN | Transcription factor AP-1 |
| | JUNB | Transcription factor jun-B |
| | PIM1 | Proto-oncogene serine/threonine-protein kinase |
| | PTPN6 | Pim-1 |
| | SOCS3 | Tyrosine-protein phosphatase non-receptor |
| | | type 6 |
| | | Suppressor of cytokine signaling 3 |
| Immune response: IL-2 | CISH | Cytokine-inducible SH2-containing protein |
| activation | EGR1 | Early growth response protein 1 |
| and signaling pathway | FOSB | Protein fosB |
| | GAB2 | GRB2-associated-binding protein 2 |
| | JUN | Transcription factor AP-1 |
| | JUNB | Transcription factor jun-B |
| | MYC | Myc proto-oncogene protein |
| | NFKBIE | NF-kappa-B inhibitor epsilon |
| | PIK3CA | Phosphatidylinositol-4,5-bisphosphate 3-kinase |
| | | catalytic subunit alpha isoform |
| | PTPN6 | Tyrosine-protein phosphatase non-receptor |
| | RELB | type 6 |
| | SOCS3 | Transcription factor ReIB |
| | | Suppressor of cytokine signaling 3 |

The number of pathways significantly induced exclusively by menadione was twice as large, 24, and included responses to extracellular responses transcription and cell cycle processes (Table 2). Hierarchal clustering of the expression of these 297 genes over all time points (Figure 1 E) showed that H2O2 and menadione-induced gene expression modification clustered completely apart.

**Table 2: Significant pathways (p<0.01) and genes induced by superoxide anion radical formation.**

| **Pathway** | **Gene Symbol** | **Gene description** |
|---|---|---|
| Signal transduction: Activin | H2BFS | Histone H2B type F-S |
| A signaling regulation | HIST1H2AC | Histone H2A type 1-C |
| | HIST1H2AD | Histone H2A type 1-D |
| | HIST1H2BB | Histone H2B type 1-B |
| | HIST1H2BC | Histone H2B type 1-C/E/F/G/I |
| | HIST1H2BE | Histone H2B type 1-C/E/F/G/I |
| | HIST1H2BF | Histone H2B type 1-C/E/F/G/I |
| | HIST1H2BG | Histone H2B type 1-C/E/F/G/I |
| | HIST1H2BH | Histone H2B type 1-H |
| | HIST1H2BI | Histone H2B type 1-C/E/F/G/I |
| | HIST1H2BL | Histone H2B type 1-L |
| | HIST1H2BM | Histone H2B type 1-M |
| | HIST1H2BN | Histone H2B type 1-N |
| | HIST1H2BO | Histone H2B type 1-O |
| | HIST1H4A | Histone H4 |
| | HIST1H4F | Histone H4 |
| | HIST1H4H | Histone H4 |
| | HIST1H4L | Histone H4 |
| | HIST2H2AC | Histone H2A type 2-C |
| | INHBB | Inhibin beta B chain |
| | SMAD3 | Mothers against decapentaplegic homolog 3 |
| | UBB | Ubiquitin |
| Vitamin B7 (biotin) | H2BFS | Histone H2B type F-S |
| metabolism | HIST1H2AC | Histone H2A type 1-C |
| | HIST1H2AD | Histone H2A type 1-D |
| | HIST1H2BB | Histone H2B type 1-B |
| | HIST1H2BC | Histone H2B type 1-C/E/F/G/I |
| | HIST1H2BE | Histone H2B type 1-C/E/F/G/I |
| | HIST1H2BF | Histone H2B type 1-C/E/F/G/I |
| | HIST1H2BG | Histone H2B type 1-C/E/F/G/I |
| | HIST1H2BH | Histone H2B type 1-H |
| | HIST1H2BI | Histone H2B type 1-C/E/F/G/I |
| | HIST1H2BL | Histone H2B type 1-L |
| | HIST1H2BM | Histone H2B type 1-M |
| | HIST1H2BN | Histone H2B type 1-N |
| | HIST1H2BO | Histone H2B type 1-O |
| | HIST2H2AC | Histone H2A type 2-C |
| | HLCS | Biotin--protein ligase |
| Cell cycle: Chromosome | CCNB2 | G2/mitotic-specific cyclin-B2 |
| condensation in | HIST1H1B | Histone H1.5 |
| prometaphase | HIST1H1E | Histone H1.4 |
| | NCAPD2 | Condensin complex subunit 1 |
| | NCAPG | Condensin complex subunit 3 |
| | NCAPH | Condensin complex subunit 2 |
| | TOP1 | DNA topoisomerase 1 |
| | TOP2A | DNA topoisomerase 2-alpha |
| | TOP2B | DNA topoisomerase 2-beta |
| Development: NOTCH1- | HDAC2 | Histone deacetylase 2 |
| mediated pathway for NF- | HIST1H4A | Histone H4 |
| KB activity modulation | HIST1H4F | Histone H4 |
| | HIST1H4H | Histone H4 |
| | HIST1H4L | Histone H4 |
| | JAG1 | Protein jagged-1 |
| | NCOR2 | Nuclear receptor corepressor 2 |
| | NCSTN | Nicastrin |
| | NFKB1 | Nuclear factor NF-kappa-B p105 subunit |
| | NFKBIE | NF-kappa-B inhibitor epsilon |
| | RELB | Transcription factor ReIB |
| Cell cycle: Start of DNA | CCNE1 | G1/S-specific cyclin-E1 |
| replication in early S phase | CDC45L | CDC45-related protein |
| | CDT1 | DNA replication factor Cdt1 |
| | E2F1 | Transcription factor E2F1 |
| | HIST1H1B | Histone H1.5 |
| | HIST1H1E | Histone H1.4 |
| | MCM3 | DNA replication licensing factor MCM3 |
| | MCM5 | DNA replication licensing factor MCM5 |
| | MCM6 | DNA replication licensing factor MCM6 |
| | RPA1 | Replication protein A 70 kDa DNA-binding |
| | | subunit |
| DNA damage: ATM/ATR | CCND1 | G1/S-specific cyclin-D1 |
| regulation of G1/S | CCNE1 | G1/S-specific cyclin-E1 |
| checkpoint | MDC1 | Mediator of DNA damage checkpoint protein 1 |
| | MYC | Myc proto-oncogene protein |
| | NFKBIE | NF-kappa-B inhibitor epsilon |
| | PCNA | Proliferating cell nuclear antigen |
| | RELB | Transcription factor ReIB |
| | SMC1A | Structural maintenance of chromosomes |
| | UBB | protein 1A |
| | | Ubiquitin |
| Leucune, isoleucine and | ALDH2 | Aldehyde dehydrogenase, mitochondrial |
| valine metabolism | ALDH6A1 | Methylmalonate-semialdehyde dehydrogenase [acylating], mitochondrial |
| | AUH | Methylglutaconyl-CoA hydratase, mitochondrial |
| | HADH | Hydroxyacyl-coenzyme A dehydrogenase, |
| | HMGCS2 | mitochondrial |
| | MCCC1 | Hydroxymethylglutaryl-CoA synthase, |
| | | mitochondrial |
| | PCCA | Methylcrotonoyl-CoA carboxylase subunit |
| | | alpha, mitochondrial |
| | | Propionyl-CoA carboxylase alpha chain, |
| | | mitochondrial |
| Cell cycle: Role of SCF | ANAPC1 | Anaphase-promoting complex subunit 1 |
| complex in cell cycle | ANAPC10 | Anaphase-promoting complex subunit 10 |
| regulation | CCND1 | G1/S-specific cyclin-D1 |
| | CCNE1 | G1/S-specific cyclin-E1 |
| | CDT1 | DNA replication factor Cdt1 |
| | CKS1B | Cyclin-dependent kinases regulatory subunit 1 |
| | E2F1 | Transcription factor E2F1 |
| | SMAD3 | Mothers against decapentaplegic homolog 3 |
| | UBB | Ubiquitin |
| Propionate metabolism | ALDH2 | Aldehyde dehydrogenase, mitochondrial |
| | ALDH6A1 | Methylmalonate-semialdehyde dehydrogenase |
| | | [acylating], mitochondrial |
| | HADH | Hydroxyacyl-coenzyme A dehydrogenase, |
| | HIBADH | mitochondrial |
| | PCCA | 3-hydroxyisobutyrate dehydrogenase, |
| | | mitochondrial |
| | | Propionyl-CoA carboxylase alpha chain, |
| | | mitochondrial |
| Transcription: Sin3 and | HDAC2 | Histone deacetylase 2 |
| NuRD in transcription | HIST1H4A | Histone H4 |
| regulation | HIST1H4F | Histone H4 |
| | HIST1H4H | Histone H4 |
| | HIST1H4L | Histone H4 |
| | MTA2 | Metastasis-associated protein MTA2 |
| | NCOR2 | Nuclear receptor corepressor 2 |
| | RXRA | Retinoic acid receptor RXR-alpha |
| | THRA | Thyroid hormone receptor alpha |
| DNA damage: Brca1 as a | CCND1 | G1/S-specific cyclin-D1 |
| transcription regulator | E2F1 | Transcription factor E2F1 |
| | IGF1R | Insulin-like growth factor 1 receptor |
| | MSH2 | DNA mismatch repair protein Msh2 |
| | MYC | Myc proto-oncogene protein |
| | PCNA | Proliferating cell nuclear antigen |
| | VEGFA | Vascular endothelial growth factor A |
| Cell cycle: Spindle | ANAPC1 | Anaphase-promoting complex subunit 1 |
| assembly and chromosome | ANAPC10 | Anaphase-promoting complex subunit 10 |
| separation | CCNB2 | G2/mitotic-specific cyclin-B2 |
| | DYNC1H1 | Cytoplasmic dynein 1 heavy chain 1 |
| | DYNC1LI2 | Cytoplasmic dynein 1 light intermediate chain 2 |
| | KPNB1 | Importin subunit beta-1 |
| | SMC1A | Structural maintenance of chromosomes |
| | TNPO1 | protein 1A |
| | TUBB2A | Transportin-1 |
| | TUBB2B | Tubulin beta-2A chain |
| | TUBB2C | Tubulin beta-2B chain |
| | TUBB3 | Tubulin beta-2C chain |
| | UBB | Tubulin beta-3 chain |
| | | Ubiquitin |
| Cell cycle: Sister chromatid | CCNB2 | G2/mitotic-specific cyclin-B2 |
| cohesion | HIST1H1B | Histone H1.5 |
| | HIST1H1E | Histone H1.4 |
| | PCNA | Proliferating cell nuclear antigen |
| | RFC4 | Replication factor C subunit 4 |
| | SMC1A | Structural maintenance of chromosomes |
| | TOP1 | protein 1A |
| | | DNA topoisomerase 1 |
| Glutathione metabolism | GSTA1 | Glutathione S-transferase A1 |
| | GSTA2 | Glutathione S-transferase A2 |
| | GSTA3 | Glutathione S-transferase A3 |
| | GSTA4 | Glutathione S-transferase A4 |
| | GSTA5 | Glutathione S-transferase A5 |
| | GSTM1 | Glutathione S-transferase Mu 1 |
| | GSTO1 | Glutathione transferase omega-1 |
| Development: Notch | HDAC2 | Histone deacetylase 2 |
| Signaling Pathway | HES1 | Transcription factor HES-1 |
| | HIST1H4A | Histone H4 |
| | HIST1H4F | Histone H4 |
| | HIST1H4H | Histone H4 |
| | HIST1H4L | Histone H4 |
| | JAG1 | Protein jagged-1 |
| | NCOR2 | Nicastrin |
| Cell cycle: ESR1 regulation | CCND1 | G1/S-specific cyclin-D1 |
| of G1/S transition | CCNE1 | G1/S-specific cyclin-E1 |
| | CKS1B | Cyclin-dependent kinases regulatory subunit 1 |
| | E2F1 | Transcription factor E2F1 |
| | MYC | Myc proto-oncogene protein |
| | UBB | Ubiquitin |
| | XPO1 | Exportin-1 |
| Transcription: P53 | E2F1 | Transcription factor E2F1 |
| signaling pathway | FHL2 | Four and a half LIM domains protein 2 |
| | HDAC2 | Histone deacetylase 2 |
| | HSPB1 | Heat shock protein beta-1 |
| | MAP4 | Microtubule-associated protein 4 |
| | MTA2 | Metastasis-associated protein MTA2 |
| | NFKB1 | Nuclear factor NF-kappa-B p105 subunit |
| | RELB | Transcription factor ReIB |
| | VEGFA | Vascular endothelial growth factor A |
| Cell cycle: Transition and | E2F1 | Transcription factor E2F1 |
| termination of DNA | PCNA | Proliferating cell nuclear antigen |
| replication | RFC4 | Replication factor C subunit 4 |
| | TOP1 | DNA topoisomerase 1 |
| | TOP2A | DNA topoisomerase 2-alpha |
| | TOP2B | DNA topoisomerase 2-beta |
| | UBB | Ubiquitin |
| Heme metabolism | CAT | Catalase |
| | FTL | Ferritin light chain |
| | GUSB | Beta-glucuronidase |
| | HCCS | Cytochrome c-type heme lyase |
| | TF | Serotransferrin |
| | UGT2B28 | UDP-glucuronosyltransferase 2B28 |
| Cell cycle: Initiation of | ANAPC1 | Anaphase-promoting complex subunit 1 |
| mitosis | ANAPC10 | Anaphase-promoting complex subunit 10 |
| tion | CCNB2 | G2/mitotic-specific cyclin-B2 |
| | HIST1H1B | Histone H1.5 |
| | HIST1H1E | Histone H1.4 |
| | LMNB1 | Lamin-B1 |
| | LMNB2 | Lamin-B2 |
| | MYC | Myc proto-oncogene protein |
| Triacylglycerol metabolism | ACSL1 | Long-chain-fatty-acid--CoA ligase 1 |
| | AGPAT3 | 1-acyl-sn-glycerol-3-phosphate acyltransferase |
| | AKR1C1 | gamma |
| | AKR1C4 | Aldo-keto reductase family 1 member C1 |
| | ALDH1B1 | Aldo-keto reductase family 1 member C4 |
| | GNPAT | Aldehyde dehydrogenase X, mitochondrial Dihydroxyacetone phosphate acyltransferase |
| Bile Acid Biosynthesis | AKR1C1 | Aldo-keto reductase family 1 member C1 |
| | AKR1C4 | Aldo-keto reductase family 1 member C4 |
| | ALDH1A1 | Retinal dehydrogenase 1 |
| | HSD3B1 | 3 beta-hydroxysteroid dehydrogenase/Delta 5-- |
| | | >4-isomerase type 1 |
| | HSD3B2 | 3 beta-hydroxysteroid dehydrogenase/Delta 5-- |
| | | >4-isomerase type 2 |
| Transcription: Role of Akt in | CCT2 | T-complex protein 1 subunit beta |
| hypoxia induced HIF1 | CCT5 | T-complex protein 1 subunit epsilon |
| activa | HSPA1A | Heat shock 70 kDa protein 1 |
| | HSPA1 B | Heat shock 70 kDa protein 1 |
| | PGK1 | Phosphoglycerate kinase 1 |
| | SLC2A1 | Solute carrier family 2, facilitated glucose |
| | | transporter member 1 |
| | TF | Serotransferrin |
| | VEGFA | Vascular endothelial growth factor A |

Expression patterns for the antioxidant enzymes SOD1, SOD2, CAT and CDKN1A were confirmed by RT-PCR, although higher expression levels were measured.

From these experiments we conclude that gene expression is different between H2O2 and menadione exposed cells, both at the level of expression as well as the level,of pathways. The gene expression measured by the microarray data was confirmed by the RT-PCR technique.

The invention therefore relates to a method for the identification of an active therapeutic compound for preventing or ameliorating the adverse effects of reactive oxygen species on eukaryotic cells comprising the steps of:
a) providing an assay wherein the expression of a pathway can be determined under the influence of a reactive oxygen species,
b) providing a candidate therapeutic compound,
c) determining the ability of said therapeutic compound to normalize the expression of said pathway after exposure to the reactive oxygen species or even in the presence of said reactive oxygen species,
   wherein said pathway is selected from the group consisting of Arginine metabolism, Polyamine metabolism, (L)-Arginine metabolism, IAP-proteins in apoptosis, regulation of aminoacid metabolism, WNT signaling pathway, Chromosome condensation in prometaphase, Urea cycle, ECM remodeling, Glucocorticoid receptor signaling, Initiation of mitosis, Aspartate and asparagine metabolism, Role of Akt in , hypoxia induced HIF1 activation, IL-3 activation and signaling pathway, IL-2 activation , and signaling pathway, Activin A signaling regulation, Vitamin B7 (biotin) metabolism, Chromosome condensation in prometaphase, NOTCH1-mediated pathway for NF-KB activity modulation, Start of DNA replication in early S phase, ATM/ATR regulation of G1/S checkpoint, , Leucine, isoleucine and valine metabolism, Role of SCF complex in cell cycle regulation, Propionate metabolism, Sin3 and NuRD in transcription regulation, Brca1 as a transcription regulator, Spindle assembly and chromosome separation, Sister chromatid cohesion, Glutathione metabolism, Notch Signaling Pathway, ESR1 regulation of G1/S transition, P53 signaling pathway, Transition and termination of DNA replication, Heme metabolism, Initiation of mitosis, Triacylglycerol metabolism ,Bile Acid Biosynthesis and Role of Akt in hypoxia induced HIF1 activa.

These pathways are characterized by the genes listed in tables 1 and 2. This means that the pathways comprise at least the genes listed in tables 1 and 2, preferably the pathways consist of the genes listed in tables 1 and 2. It is concluded that the expression of a pathway is normalized when the expression of at least one gene in the pathway reaches its normal value, preferably two or more such as more than half of the gene in the pathway, up to all of the genes in the pathway. The term "normal value" or "normalized" in this context is to be interpreted as the value obtained by the above method when the cell is not exposed to the reactive oxygen species, i.e. normal conditions.

More in particular, the invention relates to a method as described above wherein the expression of individual genes in the pathway is determined and wherein the ability of the therapeutic compound to normalize the expression of the majority of said genes is determined.

Using the criteria described in the examples, 297 overlapping genes were found for both H2O2 and Menadione exposure. A summary of significantly (p<0.01) regulated pathways and related cellular processes as indicated by MetaCore is shown in table 3.

**Table 3 Significantly regulated pathways induced by the common 297 genes.**

| **Pathway** | **Cellular Process** |
|---|---|
| Vitamin B7 (biotin) metabolism | |
| PPAR regulation of lipid metabolism | Regulation of lipid metabolism |
| Role of Akt in hypoxia induced HIF1 activation | Transcription |
| Anti-apoptotic TNFs/NF-kB/IAP pathway | Apoptosis |
| WNT signaling pathway. Part 2 | Response to extracellular stimulus |
| Regulation of lipid metabolism via LXR, NF-Y and SREBP | Transcription |
| Nitrogen metabolism | Aminoacid metabolism |
| Role of IAP-proteins in apoptosis | Transcription |
| Anti-apoptotic TNFs/NF-kB/Bcl-2 pathway | Apoptosis |
| Activin A signaling regulation | Response to extracellular stimulus |
| RXR-dependent regulation of lipid metabolism via PPAR, RAR and VDR | Transcription |
| Transcription regulation of aminoacid metabolism | Transcription |
| CDC42 in cellular processes | Small GTPase mediated signal transduction |

The number of significantly (p<0.01) regulated pathways after H2O2 exposure (Table 4A) is highest at the earlier time points, up to 1 hour. This is in accordance with the pattern of time-dependent formation of the 8-oxodG formation. Immune response was one of the most abundant regulated pathways over all time points, next to amino-acid metabolism, cell cycle, apoptosis and cell adhesion pathways. Particularly interesting is the early presence of the WNT pathway at 0 and 4 hours, taken into account that this is important in colon cancer development. Compared to H2O2, menadione resulted in more pathways that were more constantly enhanced over all time points (Table 4B). Pathways that regulate the cell cycle were most abundantly changed at all time points. Both the activin A signaling regulation pathway and vitamin B7 (biotin) metabolism was highly influenced at almost all investigated time points.

From these experiments we conclude that gene and pathway expression responses towards H2O2 was immediate, but dampened after 30 minutes, although cell cycle pathways and immune response carries on. In contrast, menadione exposure resulted in continuous change in gene and pathway expression that is not restored in 24 hours.

The invention therefore relates to a method as described above, wherein step c is conducted at different time points. In a preferred embodiment of the method, the reactive oxygen species is hydrogen peroxide or Menadione. In an also preferred embodiment, the eukaryotic cells are Caco cells, in particular Caco-2 cells.

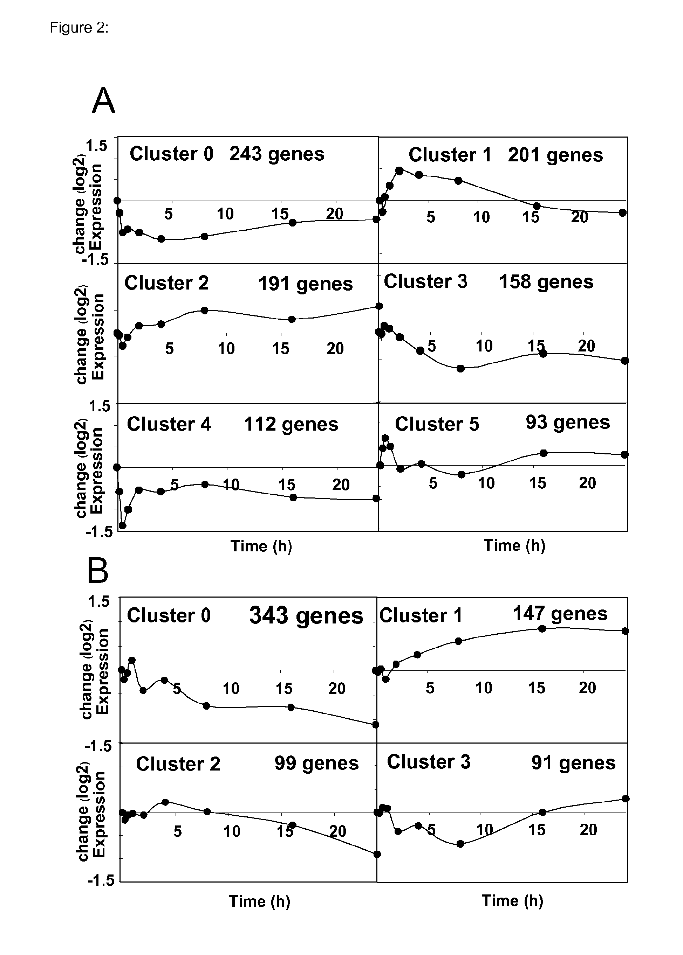

Temporal differences in gene expression were further investigated with STEM [18]. H2O2 exposure resulted in 998 genes that were significantly assigned to 6 different clusters of gene expression curves (Fig 2A). Clusters of genes were identified that were up-regulated at early, and/or late time points (clusters 1, 2 and 5), or down-regulated (clusters 0, 3 and 4) at all time points. All clusters showed profiles characterised by an immediately up- (Fig 2A, cluster 5) or down-regulation of genes at the early time points (Fig 2A, cluster 0, 1, 2, 4). Menadione exposure resulted in 4 different clusters containing 680 different genes (Fig 2B). The clusters of genes could be divided into either completely up- or down-regulated during almost all time points (cluster 0 and 1) or down-regulated at early (cluster 3) or late time points (cluster 2). Interestingly, while all clusters of co-regulated genes for H2O2 exposure mainly showed extensive changes in gene expression immediately after exposure, the clusters identified after menadione exposure showed that expression was more induced not earlier than hours after exposure.

Specific pathways regulated by the gene clusters were identified (Table 5). For both oxidant exposures, regulation of genes which are involved in the process of cell cycle regulation were found, especially in cluster 0, 2, 3, 4 and 5 for H2O2 and cluster 1 for menadione exposure. Other clusters shared in common pathways involved in nucleotide metabolism, transcription and translation, confirming the fact that gene expression related processes are initiated. Again the activin A signaling regulation pathway and vitamin B7 (biotin) metabolism is found in clusters found for both oxidants. Interestingly, in cluster 0 after H2O2 exposure, genes involved in DNA double strand break repair were found, a process that is known to be initiated by H2O2. Also uniquely for H2O2 exposure, 1 cluster contained genes that are involved in the CXCR4 signaling pathway (cluster 1) and EGF signaling (cluster 3) pathway. Unique pathways for menadione exposure included glutathione and vitamine E metabolism (cluster 0).

STEM also offers the ability to evaluate similarities and correlations in time-dependent gene expression between different experiments. No correlating clusters were found, but a significant correlation between time-dependent expressions of 30 genes was found. Pathway analysis resulted in only 1 affected pathway, being chromosome condensation in the prometaphase as part of the cell cycle processes. This again showed that the similarities between time-dependent gene expression profiles are limited, but both oxidants share in common the ability to influence cell-cycle-related processes.

From these experiments we conclude that a comparison of the profiles of co-regulated genes by STEM estimated the difference in temporal gene expression: An immediately pulse-like response to H2O2, while menadion results into gene expression that is not restored in 24 hours. Pathway analysis of the profiles confirmed the difference in gene expression caused by both oxidants, and showed that cell cycle arrest is the only common response towards different types of oxidative stress.

STEM was also used to identify the correlation between time-dependent gene expression patterns and changes in 8-oxodG formation and in cell cycle phases. For log ratio transformed 8-oxodG levels (Table 4A and B), this analysis resulted in 8 genes for H2O2 and 3 genes for menadione exposure, with no common genes, again demonstrating that the transcriptomic responses to H2O2 and menadione over 24 hours are clearly different. A number of these genes differentially expressed after H2O2 treatment is reported to be under control, or involved, in cellular oxidative stress processes. Cytochrome b5 belongs to a family of electron transport haemoproteins and is involved in the lipid peroxidation cycles induced by oxygen radicals. It is shown before that an inhibition of steroid 5-alpha reductase by finasteride results in enhancement of cellular H2O2 production [19]

As presented in Table 4C and D, time-dependent expression of many genes correlated with changes in the different phases of the cell cycle (S, G2/M, G1). For H2O2 exposure, the gene related to S phase, ADAMTS19, could no be related directly to cell cycle processes. Also arginine metabolism was the only pathway related to the G2/M phase and no pathways compromised the genes related to the G1 phase. In contrast, genes as well their induced pathways correlating to changes in cell cycle phases after menadione exposure were very relevant for the oxidant exposure and included P53 signaling, Notch signaling for NF-κB and vitamin E metabolism. The gene for which the expression pattern correlated to the G2/M phase is MCM5, and this minichromosome maintenance component 5 is also known as cell division cycle 46 which actively participates in the process of cell cycle. Comparison of the gene sets correlating with cell cycling for both exposures, showed that these shared 2 genes in common (CDKN1C, a cyclin-dependent kinase inhibitor and SLC2A4RG, a transcription factor) which both correlated to changes in the G1 phase for H2O2 and S phase for menadione exposure.

From these experiments we conclude that differentially expressed genes as well their induced pathways correlating to changes in 8-oxodG and cell cycle phases were relevant for these type phenotypical parameters..

Summary of the significant pathways (p<0.01) per time point in the differentially expressed genes after H2O2 (A) or menadione (B) exposure as indicated by MetaCore, including the exact number of pathways is shown in table 4A and 4B..

**Table 4A: General and specific pathways per time point.**

| **Time (hrs)** | **Number of pathways** | **Summary of pathways** |
|---|---|---|
| 0.08 | 8 | (Regulation of) aminoacid metabolism |
| | | Immune response: MIF-JAB1 and IL3 signalling |
| | | Development: Notch Signaling and WNT signaling pathway |
| 0.25 | 15 | Cell adhesion: ECM remodeling |
| | | Immune response: MIF-mediated glucocorticoid regulation Development: Glucocorticoid receptor and Notch signaling |
| | | Apoptosis and survival: Lymphotoxin-beta receptor signaling |
| | | Transcription regulation of aminoacid metabolism |
| 0.5 | 17 | Transcription: Role of Akt in hypoxia induced HIF1 activation and aminoacid metabolism |
| | | Development: Growth hormone and gluocorticoid receptor signaling |
| | | Immune response: Oncostatin M, IL2 and IL3 activation and signaling pathway |
| | | Oxidative stress: Role of ASK1 under oxidative stress |
| | | Apoptosis and survival: Role of IAP-proteins in apoptosis |
| | | Cell cycle: Nucleocytoplasmic transport of CDK/Cyclins |
| 1 | 6 | Transcription: Aminoacid metabolism |
| | | Immune response: Oncostatin M, IL 2 and IL 3 signaling |
| | | Development: Leptin and EGF signaling |
| 2 | 4 | Transcription: Aminoacid metabolism |
| | | Immune response: Oncostatin M signaling via MAPK in human cells |
| | | Cholesterol Biosynthesis |
| | | Development: EGF signaling pathway |
| 4 | 5 | Transcription: Aminoacid metabolism |
| | | Cell cycle: Chromosome condensation in prometaphase |
| | | Immune response: Oncostatin M signaling via MAPK in human cells |
| | | Development: WNT signaling pathway |
| 8 | 8 | Polyamine, arginine and biotin metabolism |
| | | Development: Glucocorticoid receptor signaling |
| | | Transcription: Role of Akt in hypoxia induced HIF1 activation |
| | | Apoptosis and survival: Role of IAP-proteins in apoptosis |
| | | Cell cycle: Initiation of mitosis |
| | | Cell adhesion: ECM remodeling |
| 16 | 5 | Arginine metabolism |
| | | Development: EPO-induced Jak-STAT pathway Urea cycle |
| | | Immune response: Antigen presentation by MHC class I |
| | | Cell adhesion: ECM remodeling |
| 24 | 6 | Arginine, polyamine, CTP/UTP and ATP/ITP metabolism |
| | | Urea cycle |
| | | Cell cycle: Initiation of mitosis |

**Table 4B**

| **Timepoint (hrs)** | **Number of pathways** | **Summary of Pathways** |
|---|---|---|
| All | | Cell cycle: various pathways |
| All except 2 hrs | | Vitamin B7 (biotin) metabolism |
| 0.08 | 18 | Signal transduction: Activin A and AKT signaling regulation |
| | | Glutathione metabolism |
| | | Cytoskeleton remodeling: CDC42 in cellular processes |
| | | Transcription: Formation of Sin3A and NuRD and |
| | | Aminoacid metabolism |
| 0.25 | 16 | Signal transduction: Activin A signaling regulation |
| | | Cytoskeleton remodeling: CDC42 in cellular processes |
| | | Transcription: Sin3A and NuRD complexes, aminoacid metabolism and role of HP1. |
| | | Development: TGF-beta receptor signaling and TPO in cell process |
| 0.5 | 14 | Transcription: Formation of Sin3A and NuRD complexes, aminoacid metabolism, androgen receptor nuclear signaling and role of HP1 |
| | | Signal transduction: Activin A signaling regulation |
| 1 | 15 | Signal transduction: Activin A and Akt signaling Glutathione and nitrogen metabolism |
| | | Cytoskeleton remodeling: CDC42 in cellular processes |
| | | Transcription: Role of Akt in hypoxia induced HIF1 activation |
| | | Development: WNT signaling pathway |
| 2 | 8 | Spindle assembly and chromosome separation |
| | | Transport: RAN regulation pathway |
| | | Transcription: Role of HP1 family in transcriptional silencing |
| 4 | 10 | Signal transduction: Activin A signaling regulation |
| | | Cytoskeleton remodeling: CDC42 in cellular processes |
| | | Transcription: Formation of Sin3A and NuRD complexes |
| | | Apoptosis and survival: Role of IAP-proteins in apoptosis |
| 8 | 14 | Signal transduction: AKT and Activin A signaling regulation |
| | | Cytoskeleton remodeling: CDC42 in cellular processes |
| | | Development: Notch Signaling Pathway and NF-kB activation |
| | | Bile Acid Biosynthesis |
| | | IMP and ATP/ITP metabolism |
| 16 | 19 | Signal transduction: Akt 1 and Activin A signaling regulation |
| | | Cytoskeleton remodeling: CDC42 in cellular processes |
| | | Transcription: Formation of Sin3A and NuRD complexes and their role in transcription regulation |
| | | Development: Notch, VEGF, IGF and Angiopoietin |
| | | Signaling |
| | | Transcription: P53 signaling pathway |
| | | Transport: Rab-9 regulation pathway |
| 24 | 18 | Amino acids, nitrogen and triacylglycerol metabolism |
| | | Signal transduction: Activin A signaling regulation |
| | | Cytoskeleton remodeling: CDC42 in cellular processes |
| | | Transcription_Formation of Sin3A and NuRD complexes and p53 Development: VEGF, Notch Signaling Pathway and NF-kB activation |
| | | Bile Acid Biosynthesis |

Significantly (p<0.01) regulated pathways as indicated by MetaCore of the gene expression profiles found by STEM are shown in figure 2. Table 5 shows a summary of the pathways and cellular processes after H2O2 (A) or menadione (B).

**Table 5A: Summary of the pathways of the gene expression cluster profiles.**

| **Cluster** | **Number** | **Pathways** | **Processes** |
|---|---|---|---|
| 0 | 4 | - Chromosome condensation in prometaphase and metaphase checkpoint | - cell cycle |
| | | | - DNA damage |
| | | - Mechanism of DSB repair | - transport |
| | | - Clatherin coated-vesicle cycle | |
| 1 | 1 | - CXCR4 signaling | - G-coupled protein signaling/immune respons |
| 2 | 9 | - ATP, CTP/UTP. GTP metabolism | - nucleotide metabolism |
| | | - Role of APC in cell cycle regulation | - cell cycle |
| | | - Aspartate, aspargine and polyamine metabolism | - metabolism |
| | | | - transcription, hypoxia |
| | | - Role of Akt in hypoxia induced HIF1 activation | |
| 3 | 1 | - EGF signaling pathway | - growth factor, development |
| 4 | 3 | - Regulation of translation initiation | - translation |
| | | - Transition and termination of DNA replication | cell cycle |
| | | | - cell cycle |
| | | - Nucleocytoplasmic transport of CDK/Cyclins | |
| 5 | 4 | - Vitamin B7 metabolism | - metabolism |
| | | - Activin A signal regulation | - cell cycle |
| | | - CDC42 in cellular processes | - cytoskeleton remodeling |
| | | - TPO signaling via JAK-STAT pathway | - development |

**Table 5B**

| **Cluster** | **Number** | **Pathway** | **Process** |
|---|---|---|---|
| 0 | 12 | - Iso(leucine), valine, trypthophane, proline metabolism | - aminoacid metabolism |
| | | - Gluthathione and vitamin E metabolism | - vitamin metabolism |
| | | - Propionate and triaglycerol metabolism, mitochondrial long chain and peroxisomal branched chain fatty acid oxidation | |
| | | | - lipid metabolism |
| | | | |
| | | - Notch signaling and activating pathway | |
| | | | - development |
| 1 | 18 | - Vitamin B7 metabolism | - vitamin metabolism |
| | | - Activin A signal regulation | |
| | | - CDC42 and ATM./ATR regulation of G1/S checkpoint | - cell cycle |
| | | | - cell cycle |
| | | - Parkin in and ubiquitin proteasomal pathway | - proteolyse |
| | | - P53, NF-κB and NGF activation of NF-κB, VEGF signalling | - transcription |
| 2 | 4 | - Cholesterol, cortisone, bile acid, androstenedione and testosterone metabolism | - metabolism |
| 3 | 8 | - WNT signaling | - development |
| | | - Activin A in cell differentiation | - development |
| | | - GTP, CTP/UTP, ATP metabolism | - nucleotide metabolism |
| | | - Endothelin-1/EDNRA signaling | |
| | | - Androgen receptor nuclear signaling | - development |
| | | | - transcription |

Table 6A shows the genes that clustered in the same gene expression cluster profiles with 8-oxodG levels or cell cycle phases. Correlating genes clustered by STEM with the 8-oxodG levels induced by exposure to 20 uM H2O2 (A) or 100 uM menadione

**Table 6A**

| **Gene symbol** | **Description** |
|---|---|
| CYB5A | cytochrome b5 type A |
| SRD5A2L | steroid 5 alpha-reductase 2-like |
| KLHL13 | kelch-like 13 |
| APH1B | anterior pharynx defective 1 homolog B |
| IFIT1 | interferon-induced protein with tetratricopeptide repeats 1 |
| SDSL | serine dehydratase-like |
| PEPD | peptidase D |
| OAT | ornithine aminotransferase, nuclear gene encoding mitochondrial protein |

Table 6B shows the genes of which the time-dependent expression is clustered by STEM with cell cycle by exposure to 20 uM H2O2.

**Table 6B**

| **Gene symbol** | **Description** |
|---|---|
| SH3BGRL3 | SH3 domain binding glutamic acid-rich protein like 3 |
| FRMD4A | FERM domain containing 4A |
| DKFZP434H132 | DKFZP434H132 protein |

Table 6C shows the genes of which the time-dependent expression is clustered by STEM with cell cycle by exposure to 100 uM menadione.

**Table 6C**

| **Cell cycle phase** | **genes** | **pathways** | **Summary of pathways** |
|---|---|---|---|
| S | 1 | 0 | - |
| G2/M | 45 | 1 | Arginine metabolism |
| G1 | 29 | 0 | - |

Table 6D including induction of significant (p<0.01) pathways as indicated by MetaCore.

**Table 6D**

| **Cell cycle phase** | **genes** | **pathways** | **Summary of pathways** |
|---|---|---|---|
| S | 74 | 4 | P53 signaling, androstenedione, testosterone and bile acid synthesis and metabolism, triaglycerol metabolism |
| G2/M | 1 | 0 | - |
| G1 | 311 | 15 | Amino acid metabolism, Notch signaling (for NF-κB), PPAR regulation of lipid metabolism, cholesterol and vitamin E metabolism, fatty acid oxidation, bile acid biosynthesis, cadherin-mediated cell adhesion |

While ESR spectrometry showed that both types of oxidants induced comparable cellular oxygen free radical levels, the kinetics of 8-oxodG formation were different. It is possible that time-dependent cellular conversion of 02.- into the much more effective DNA-oxidizing HO. radicals resulted in this highest level of 8-oxodG 2 hours after menadione exposure. Extensive studies to the time-dependent formation 8-oxodG by H2O2 or menadione in caco-2 cells were not published before. Only one study with menadione in rat hepatocytes reported the absence of 8-oxodG formation in rat hepatocytes up to 2 hours after incubation [20]. Analysis of cell cycle effects indicated a similar temporal difference between H2O2 and menadione. Altogether this shows that the formation of oxidative DNA damage by both oxidants is followed by cell cycle arrest, but both effects are induced at a slower rate after the increase of intracellular 02.-.

This difference in temporal response to both oxidants is reflected at the gene expression level: Analysis of the time-dependent co-regulated genes revealed that the cells responded immediately on HO. by an early up- or down-regulation of genes at early time points. Genes in these clusters are specifically involved in transcription, cell cycle, cell differentiation and metabolic processes. But uniquely for H2O2 exposure, genes involved in DNA double strand break repair, CXCR4 signaling pathway and EGF signaling pathway were found. The chemokine receptor CXCR4 is constitutively expressed in caco-2 cells [21], involved in colon carcinogenesis and proposed as a prognostic factor for gastrointestinal tumor formation [22]. The epidermal growth factor (EGF) receptor is tyrosine kinase receptor involved in growth, proliferation and apoptosis signaling of cells and activation is observed in gastrointestinal carcinogenesis.

In contrast, an increase in intracellular O2.- caused gradual increase in up- or down regulated genes up to 24 hours, while again, cell cycle and metabolic processes are biological functions in these clusters. Unique pathways for menadione exposure included glutathione and vitamine E metabolism, pathways involved in the antioxidant response towards oxidative stress. Altogether, the difference in gene expression patterns for both oxidants explains the separation of the exposures in the hierarchal clustering of the 297 commonly regulated genes. The pulse-like response towards H2O2, including recovery within 1 hour, while no recovery was observed with menadione, is completely comparable to the gene expression effects previously described for the fungus Aspergillus nidulans [23]. Comparison of pathways showed that (secondary) metabolism also was found to be induced by both oxidants in this study.

The sequential effects caused by the oxidants are revealed in detail by pathway analysis of the gene list per time point. Within 5 minutes after H2O2 exposure, the cells responded to oxidative stress by showing an immune-like response, via macrophage migration inhibitory factor (MIF)-JAB1 and IL3. The cytokine MIF interacting with JAB1, antagonizes cell-cycle regulation [24], while IL-3 modulates colon cancer cell growth in vitro [25]. The cell cycle pathway indeed appears hereafter. MIF regulation is also seen 15 minutes after H2O2 exposure. Also Notch signalling is immediately induced and this signalling network is involved in colon carcinogenesis [26] and ageing [27]. After 30 minutes, apoptosis signal-regulating kinase 1 (ASK1) is affected, being a MAP kinase kinase kinase preferentially activated in response to oxidative stress and plays pivotal roles in a wide variety of cellular responses [28]. After 30 minutes, gene and pathway expression responses dampen, although cell cycle pathways and immune response, especially via oncostatin M signalling, carries on. Oncostatin M is related to IL31 induction and seen in inflammatory bowel diseases [29]. In contrast to the pulse-like modulation of gene and pathway expression profiles, amino-acid metabolism is a pathway that is continuously affected over 24 hour.

Menadione exposure continuously modulates the cell cycle pathways and vitamin B7 (biotin) metabolism. This vitamin is a prosthetic group for carboxylases, plays a role in lipid metabolism, and biotin is via biotinylation a function as a keeper of gene expression. Deficiency of biotin results in increased mitochondrial ROS formation via an impaired respiration [30]. Further, Activin A is almost continuously present in the signal transduction pathway. Activin is a pro-inflammatory mediator, and overexpressed in gastrointestinal inflammation [31]. In contrast to H2O2 exposure however, no (immediate) responses of immune pathways or pathways specifically assigned to oxidative stress were revealed. Interestingly, H2O2 immediately and menadione after 1 hour induced the WNT signalling pathway, which is a key player in colon carcinogesis [32].

Microarray analysis of the effect of 75 µM H2O2 on the expression of 12931 genes in Caco-2 cells at 30 minutes incubation time was done before [33] and this resulted in the selection of 87 affected genes. Comparison of the genes showed 29 genes (33%) are similar if leaving out specific subtypes of proteins. At the functional level identical pathways, i.e. cell development and cell cycle were identified if compared with our results at T=0.5h. Comparison with the time-dependently induced probe set found after exposing human A549 lung cells to the H2O2-generating system [34] showed that 12 out of 51 genes (24%) were similar, of which most genes are involved in cell cycle processes.

Genes as well their induced pathways correlating to changes in 8-oxodG and cell cycle phases were relevant for these type phenotypical parameters. Comparison of both gene sets for the cell cycle revealed the presence of a cyclin-dependent kinase inhibitor (CDKN1C) that is clearly involved in the regulation of cell cycle processes.

Altogether, we now report as a novel finding that although a large number of identical genes were affected by both oxidants, no similarity in the temporal expression of these genes was found. Comparison of the profiles of co-regulated genes showed that gene expression in Caco-2 cells immediately reacted by a pulse-like response to the HO. formation, while formation of 02.- results into gene expression that is not restored in 24 hours. Pathway analyses identified the sequential involvement of gene expression in various cell responses and demonstrated that the difference in the modulation of gene expression is also reflected in regulation of pathways by HO. and O2.-: H2O2 immediately influences pathways involved in the immune function, while menadione constantly regulated cell cycle-related pathways. Importantly, temporal effects of HO. and 02.- can be discriminated regarding their modulation of particular carcinogenesis-related mechanisms. Altogether, the temporal response of Caco-2 cells towards two oxidants is different at all levels of gene expression: temporal patterns, influenced pathways and phenotypical anchoring, clearly demonstrating that HO. and 02.- need to be differentially associated with oxidative stress-related colon diseases including cancer.

This strongly emphasizes that for the biological interpretation of genome wide gene expression, studying time-dependent effects is inevitable. Taken into account that the cellular defence capacity against oxidative stress in the human body is strongly influenced by the uptake of dietary antioxidants in the colon, it would be interesting to translate the oxidants-induced temporal gene expression profiles into the beneficial health effect of antioxidants in food on colon cells. For example, a comparison showed that the genes MT1 G, CCNG1, ATF3 and ODC1 are in both differentially expressed by oxidative stress in Caco-2 cells and in colonic tissue of colorectal patients after intake of a high vegetable-containing diet [35,36]

### References

1. Bruce,W.R., et al., (2002) IARC Sci Publ, 156, 277-281.
2. Hussain,S.P., et al., (2003) Nat Rev Cancer, 3, 276-285.
3. Sanders,L.M., et al., (2004) Cancer Lett, 208, 155-161.
4. Pavlick,K.P., et al., (2002) Free Radic Biol Med, 33, 311-322.
5. Itzkowitz,S.H. and Yio,X. (2004) Am J Physiol Gastrointest Liver Physiol, 287, G7-17.
6. Winterbourn,C.C. and Metodiewa,D. (1999) Free Radic Biol Med, 27, 322-328.
7. D'Autreaux,B. and Toledano,M.B. (2007) Nat Rev Mol Cell Biol, 8, 813-824.
8. Dinkova-Kostova,A.T., et al., (2005) Chem Res Toxicol, 18, 1779-1791.
9. Kobayashi,M. and Yamamoto,M. (2006) Adv Enzyme Regul, 46, 113-140.
10. Morel,Y. and Barouki,R. (1999) Biochem J, 342, 481-496.
11. Slupphaug,G., et al., (2003) Mutat Res, 531, 231-251.
12. Clopton,D.A. and Saltman P. (1995) Biochem Biophys Res Commun, 210, 189-196.
13. Briedé,J.J., et al., (2005) FEMS Immunol Med Microbiol, 44, 227-232.
14. Staal,Y.C., et al., (2007) Carcinogenesis, 28, 2632-2640.
15. Briedé,J.J., et al., (2004) Free Radic Res, 38, 995-1002.
16. Livak,K.J. and Schmittgen,T.D. (2001) Methods, 25, 402-408.
17. Reich,M., et al., (2006) Nat Genet, 38, 500-501.
18. Ernst,J. and Bar-Joseph,Z. (2006) BMC Bioinformatics, 7, 191.
19. Cayatte,C., et al., (2006) Mol Cell Proteomics, 5, 2031-2043.
20. Fischer-Nielsen,A., et al., (1995) Biochem Pharmacol, 49, 1469-1474.
21. Dwinell,M.B., et al., (2004) Am J Physiol Gastrointest Liver Physiol, 286, G844-850.
22. Schimanski,C.C., et al., (2008). World J Gastroenterol, 14, 4721-4724.
23. Pocsi,l., et al., (2005) BMC Genomics, 6, 182.
24. Kleemann,R., et al., (2000) Nature, 408, 211-216.
25. Block,T., et al., (1992) Urol Res, 20, 289-292.
26. Katoh,M. and Katoh,M. (2007) Int J Oncol, 30, 247-251.
27. Carlson,M.E., et al., (2008) Exp Cell Res, 314, 1951-1961.
28. Matsuzawa,A. and Ichijo,H. (2008) Biochim Biophys Acta, 1780, 1325-1336.
29. Dambacher,J., et al., (2007) Gut, 56, 1257-1265.
30. Depeint,F., et al., (2006) Chem Biol Interact, 163, 94-112.
31. Werner,S. and Alzheimer,C. (2006) Cytokine Growth Factor Rev, 17, 157-171.
32. Gmeiner,W.H., et al., (2008) Oncol Rep, 19, 245-251.
33. Herring, T.A., et al., (2007) Dig Dis Sci, 52, 3005-15.
34. Dandrea,T., et al., (2004) Free Radic Biol Med, 36, 881-896.
35. van Breda,S.G., et al., (2004) Carcinogenesis, 25, 2207-2216.
36. van Breda,S.G., et al., (2005) J Nutr, 135, 1879-1888.

### Examples

### Example 1:Cell culture and treatment

The Caco-2 human colorectal adenocarcinoma cell line (American Type Culture Collection, Manassas, VA, USA) was grown in Dulbecco's Modified Eagle's Medium supplemented with 10% fetal bovine serum, 1% non-essential amino acids, 1% sodium pyruvate and 1% penicillin/streptomycin, at 37°C, and 5% CO2. Cells were grown until 80% confluency before medium was replaced by DMEM without supplements containing 100 µM menadione or 20 µM H2O2. For microarray analyses, time-matched control cells were treated in an identical manner without addition of the oxidants. Samples were taken after 0.08, 0.25, 0.5, 1, 2, 4, 8, 16, or 24 hours. Because ESR measurements showed radical scavenging activity by the supplements, these were supplied 45 minutes after oxidant addition. At the indicated time points, cells were fixed in 2 ml ice cold methanol for flow cytometry or medium was replaced by 1 ml Trizol for RNA/DNA isolation.

### Example 2: Electron spin resonance (ESR) spectroscopy

Caco-2 cells were washed with PBS, pre-incubated for 30 minutes with 50 mM DMPO, and washed with PBS again. After exposure to H2O2 or menadione as described above, cells were scraped. ESR spectra from cells were recorded on a Bruker EMX 1273 spectrometer with instrumental conditions and quantification of DMPO.-OH peak signals as described before [13].

### Example 3:Flow cytometric analyses

Analyses of cell cycle profiles and apoptosis was performed as previously described [14]. Cells were stained with propidium iodide; apoptotic cells were visualised by means of the primary antibody M30 CytoDeath (Roche, Penzberg, Germany) and FITC conjugated anti-mouse Ig as secondary antibody (DakoCytomation, Glostrup, Denmark). Data analysis was done using CellQuest software (version 3.1, Becton Dickinson, San Jose, USA). M30 CytoDeath positive (apoptotic) and negative (non-apoptotic) signals were displayed as percentage of total cells with WinMDI 2.8 (http://facs.scripps.edu/software.html). Cell cycle profiles were analyzed using ModFit LT for Mac (version 2.0).

### Example 4: RNA and DNA isolation

RNA was isolated from the Trizol solutions according to the producer's manual and purified with the RNeasy mini kit (Qiagen Westburg bv., Leusden, The Netherlands). After removal of the aqueous phase during RNA isolation using Trizol, remaining phases were used for DNA isolation according to manufacturer's protocol. RNA and DNA quantity was measured on a spectrophotometer and RNA quality was determined on a BioAnalyzer (Agilent Technologies, Breda, The Netherlands). Only RNA samples which showed clear 18S and 28S peaks and with a RIN>8 were used.

### Example 5: 8-oxodG analyses

Analysis of 8-oxo-dG was performed as described earlier [15]. Briefly, after extraction, DNA was digested into deoxyribonucleosides by treatment with nuclease P1 [0.02 U/µl] and alkaline phosphatase [0.014 U/µl]. The digest was then injected into a HPLC-ECD-system. The mobile phase consisted of 10% aqueous methanol containing 94 mM KH2PO4, 13 mMK2HPO4, 26 mM NaCl and 0.5 mM EDTA. The detection limit was 1.5 residues/1 06 2'-deoxyguanosine (dG). UV-absorption of dG was simultaneously monitored at 260 nm.

### Example 6: Real-time polymerase chain reaction

Quantitative real-time polymerase chain reaction (RT-PCR) was performed as reported [14]. All PCR reactions were performed in duplicate. β-actin messenger RNA was used as reference. The RT-PCR was run on the MyiQ Single-Color Real-Time PCR Detection System (Bio-Rad Laboratories). The following forward and reverse primers were used (OPERON, 5'-3' sequences): SOD1 GTGGTCCATGAAAAAGCAGATGA (forward) and CACAAGCCAAACGACTTCCA (reverse), SOD2 ATCAGGATCCACTGCAAGAA (forward) and CGTGCTCCCACACATCAATC (reverse), catalase GACTGACCAGGGCATCAAAAA (forward) and CGGATGCCATAGTCAGGATCTT (reverse) and β-actin CCTGGCACCCAGCACAAT (forward) and GCCGATCCACACGGAGTACT (reverse). Expression of selected genes is calculated, using the DeltaDeltaCt log method and log base 2 transformed [16].

### Example 7: Microarrays, labelling and hybridization

Labelling and hybridisation of RNA samples were done according to Agilent's manual for microarrays with minor modifications ((Agilent Technologies, Breda, The Netherlands). RNA (0.5µg) was transcribed into cDNA and labelled with Cyanine 3 (Cy3) or Cyanine 5 (Cy5). When dye-incorporation was above 7 pmol/µg RNA, 2 µg cRNA of the treated and time-matched control samples was applied on G4110B 22K and G4112F 4x44K Agilent Human Oligo Microarray. Slides were scanned on a GenePix 4000B (Molecular Devices, Sunnyvale, USA) with fixed laser power (100%) and PMT gain. For each exposure, two biological repeats per time point were performed. Also swapped Cy3 and Cy5 dye labelling was done, resulting in 72 hybridisations.

### Example 8: Microarrays, image analysis and processing

Images were processed with ImaGene 6.0 software (BioDiscovery Inc., Los Angeles, USA) to quantify spot signals. Irregular spots were flagged. Data from ImaGene were transported into GeneSight software version 4.1.6 (BioDiscovery Inc., Los Angeles, USA). For each spot background was subtracted and flagged spots as well as spots with a net expression level below 20 in both channels were omitted. Data were log base 2 transformed and LOWESS normalization was applied. Expression difference with the time-matched control was calculated and if more than one probe of a gene was present on the array, the replicates were combined while omitting outliers (>2 standard deviations). Only the probe sets present at both types of array platforms were selected.

### Example 9: Gene expression data analyses; differentially expressed genes

Genes were selected for which all four replicate hybridizations (2 biological experiments with 2 hybridisations) resulted in a log base 2 expression difference of >0.5 or all four <-0.5 (all replicates in the same direction). The webtool GenePattern [17] (http://genepattern.broad.mit.edu/gp/pages/index.jsf) was used for clustering and visualization of heat maps and cluster patterns.

### Example 10: Time series analyses

For identification of genes co-regulated time-dependently and clustering with the phenotypic parameters, the software tool "Short Time-series Expression Miner" (STEM, version 1.1.2b; http://www.cs.cmu.edu/∼jernst/stem/) was used [18]. The clustering algorithm assigns each gene passing the filtering criteria (maximally 4 missing values, minimum correlation between experiment 1 and 2 of 0.3) to these profiles based on correlation coefficients and permutation analyses (n=50). Profiles are clustered by correlation analyses. For clustering 8-oxodG and cell cycle distribution levels were transformed into log 2 base ratios.

### Example 11: Functional interpretation of significantly differentially expressed gene sets

MetaCore™ (GeneGo, San Diego, CA) was used to identify and visualize the involvement of the differentially expressed genes in the biological processes that may be affected at the level of pathways, by selecting significant pathways with a p-value<0.01.

### Example 12: Statistical analyses 8-oxodG levels and cell cycle effects

Data are presented as means ± SD. Statistical analyses of changes in apoptosis, 8-oxodG levels or cell cycle phases were performed using SPSS for Windows 14.0 software (SPSS Inc., Chicago, IL, USA) using a students t-test with statistical significance set at p<0.05.

### Legend to the figures

Figure 1: Levels of ratio of 8-oxodG/dG (A) and cell cycle distribution of Caco-2 cells after exposure to 20 uM H2O2 (B) or 100 uM Menadione (C). Venn diagram (D) showing overlapping and unique genes in the differential expressed gene data sets after the exposure to 20 µM H2O2 or 100 uM Menadione. The genes present in different clusters are described in the supplementary data Table 1. Dendogram (E) of the result of the hierarchal clustering of the identical genes in the data set after exposure to 20 uM H2O2 (H2O2) or 100uM Menadione (Men) over all time points.

Figure 2 Average expression curves for the clusters of genes generated by STEM from Caco-2 cells after various exposure times to 20 µM H2O2 (A) or 100 µM Menadione (B).

## Claims

1. Method for the identification of an active therapeutic compound for preventing or ameliorating the adverse effects of reactive oxygen species on eukaryotic cells comprising the steps of:
a. providing an assay wherein the expression of a pathway can be determined under the influence of a reactive oxygen species,
b. providing a candidate therapeutic compound,
c. determining the ability of said therapeutic compound to normalize the expression of said pathway after exposure to said reactive oxygen species or even in the presence of said reactive oxygen species,
wherein said pathway is selected from the group consisting of consisting of Arginine metabolism, Polyamine metabolism, (L)-Arginine metabolism, IAP-proteins in apoptosis, regulation of aminoacid metabolism, WNT signaling pathway, Chromosome condensation in prometaphase, Urea cycle, ECM remodeling, Glucocorticoid receptor signaling, Initiation of mitosis, Aspartate and asparagine metabolism, Role of Akt in , hypoxia induced HIF1 activation, IL-3 activation and signaling pathway, IL-2 activation , and signaling pathway, Activin A signaling regulation, Vitamin B7 (biotin) metabolism, Chromosome condensation in prometaphase, NOTCH1-mediated pathway for NF-KB activity modulation, Start of DNA replication in early S phase, ATM/ATR regulation of G1/S checkpoint, , Leucine, isoleucine and valine metabolism, Role of SCF complex in cell cycle regulation, Propionate metabolism, Sin3 and NuRD in transcription regulation, Brca1 as a transcription regulator, Spindle assembly and chromosome separation, Sister chromatid cohesion, Glutathione metabolism, Notch Signaling Pathway, ESR1 regulation of G1/S transition, P53 signaling pathway, Transition and termination of DNA replication, Heme metabolism, Initiation of mitosis, Triacylglycerol metabolism ,Bile Acid Biosynthesis and Role of Akt in hypoxia induced HIF1 activa.

2. Method according to claim 1 wherein the expression of individual genes in the pathway is determined and wherein the ability of the therapeutic compound to normalize the expression of the majority of said genes is determined.

3. Method according to claims 1 or 2 wherein step c is conducted at different time points

4. Method according to claims 1 - 3 wherein the reactive oxygen species is hydrogen peroxide or Menadione.

5. Method according to claims 1 - 4 wherein the eukaryotic cells are Caco cells, in particular Caco-2 cells.


SEQUENCE LISTING

<110>  Universiteit Maastricht

<120>  METHOD FOR SCREENING COMPOUNDS FOR PREVENTING THE ADVERSE EFFECTS
        OF REACTIVE OXYGEN SPECIES ON EUKARYOTIC CELLS

<130>  071 EPPO

<160>  8

<170>  PatentIn version 3.5

<210>  1
<211>  23
<212>  DNA
<213>  Artificial Sequence

<220>
<223>  Probes and primers

<400>  1
gtggtccatg aaaaagcaga tga                                                23

<210>  2
<211>  20
<212>  DNA
<213>  Artificial Sequence

<220>
<223>  Probes and primers

<400>  2
cacaagccaa acgacttcca                                                    20

<210>  3
<211>  20
<212>  DNA
<213>  Artificial Sequence

<220>
<223>  Probes and primers

<400>  3
atcaggatcc actgcaagaa                                                    20

<210>  4
<211>  20
<212>  DNA
<213>  Artificial Sequence

<220>
<223>  Probes and primers

<400>  4
cgtgctccca cacatcaatc                                                    20

<210>  5
<211>  21
<212>  DNA
<213>  Artificial Sequence

<220>
<223>  Probes and primers

<400>  5
gactgaccag ggcatcaaaa a                                                  21

<210>  6
<211>  22
<212>  DNA
<213>  Artificial Sequence

<220>
<223>  Probes and primers

<400>  6
cggatgccat agtcaggatc tt                                                22


<210>  7
<211>  18
<212>  DNA
<213>  Artificial Sequence

<220>
<223>  Probes and primers

<400>  7
cctggcaccc agcacaat                                                     18


<210>  8
<211>  20
<212>  DNA
<213>  Artificial Sequence

<220>
<223>  Probes and primers

<400>  8
gccgatccac acggagtact                                                   20